## Europäisches Patentamt

## European Patent Office

## Office européen des brevets

(11) Veröffentlichungsnummer: **0 269 048 B1**

## EUROPÄISCHE PATENTSCHRIFT

(45) Veröffentlichungstag der Patentschrift: 31.07.91

(51) Int. Cl.⁵: **A61B 7/02**, A61B 9/00, A61B 3/00

(21) Anmeldenummer: 87117220.1

(22) Anmeldetag: 23.11.87

(54) Stethoskop.

(30) Priorität: 21.11.86 DE 8631201 U

(43) Veröffentlichungstag der Anmeldung:
01.06.88 Patentblatt 88/22

(45) Bekanntmachung des Hinweises auf die
Patenterteilung:
31.07.91 Patentblatt 91/31

(84) Benannte Vertragsstaaten:
AT BE CH DE ES FR GB GR IT LI LU NL SE

(56) Entgegenhaltungen:
CH-A- 319 404
DE-U- 8 631 201
FR-A- 563 469
US-A- 2 532 093

(73) Patentinhaber: **Gerding, Hartmut**
**Voltastrasse 65**
**W-4150 Krefeld(DE)**

Patentinhaber: **Hachenberg, Günter**
**Untere Metzgerstrasse 22**
**W-5900 Siegen(DE)**

(72) Erfinder: **Gerding, Hartmut**
**Voltastrasse 65**
**W-4150 Krefeld(DE)**
Erfinder: **Hachenberg, Günter**
**Untere Metzgerstrasse 22**
**W-5900 Siegen(DE)**

(74) Vertreter: **Grosse, Dietrich, Dipl.-Ing. et al**
**Patentanwälte HEMMERICH-**
**MÜLLER-GROSSE-POLLMEIER-MEY Hammer-**
**strasse 2**
**W-5900 Siegen 1(DE)**

Rank Xerox (UK) Business Services

## Beschreibung

Die Erfindung betrifft ein Stethoskop mit einem Rumpf 1 mit mindestens einer trichterförmigen Ausnehmung zum Erfassen des Schalles und mit zwei mit dem Grunde der Ausnehmung in Verbindung stehenden, durch Ohroliven abgeschlossenen Schläuchen, bei dem der Rumpf durch eine elastische Umhüllung oder durch mindestens einen elastischen Hammeraufsatz zu einem Hammerkopf ausgebildet ist, der mit einem rohrförmigen Griff verbunden oder verbindbar ist. Derartige Schlauch-Sethoskope dienen zum Abhören des Körpers eines Patienten mit beiden Ohren des Diagnostizierenden zur Erfassung von bspw. Herztönen oder Atemgeräuschen des Patienten.

Es ist bei Ärzten allgemein üblich, bspw. während der Visiten einige ärztliche Geräte in den Taschen des Arztkittels mit sich zu tragen. Hierbei besteht jedoch die Gefahr, daß beim Beugen über den Patienten oder dergleichen Taschen sich ungewollt entleeren können. Zu den ärztlichen Geräten, die am häufigsten verwendet werden, gehört bspw. ein Stethoskop, das zur Auskulation, d.h., zum Abhorchen des Körpers des Patienten, verwendet wird. Ferner wird zur Überprüfung von Reflexen ein Gummihammer und/oder eine Reflexnadel benötigt, und eine Lichtquelle wird nicht nur zur Ausleuchtung benutzt, sondern findet auch bspw. zur Überprüfung von Augenreflexen Verwendung. Schon beim Mitführen dieser Geräte bietet deren Unterbringung Schwierigkeiten, und beim Eintritt einer Visite ist nicht ohne weiteres festzustellen, ob auch alle benötigten Geräte mitgeführt werden. Weiterhin besteht bei der Mitnahme der Geräte stets die Gefahr, daß eines derselben verloren werden kann, ohne daß dieses sofort bemerkt wird. Einzig das Stethoskopf wird im allgemeinen gut sichtbar und bemerkbar getragen.

In der CH-A-319 404 wird ein Stethoskop beschrieben, das durch Umbildung seines Rumpfes zu einem Hammerkopf auch gleichzeitig zum Auskultieren verwendbar ist, und das zusätzlich über eine entnehmbare Reiznadel verfügt.

Die Erfindung geht daher von der Aufgabe aus, die wesentlichen, bei Krankenbesuchen und Visiten stets mitzuführende Geräte zu einem raumsparenden Universalgerät zusammenzufassen, so daß bei einfacher Unterbringung durch geringen Platzbedarf Verluste einzelner Geräte weitgehend ausgeschlossen werden und ihre Vollzähligkeit gesichert ist.

Gelöst wird diese Aufgabe, indem der Griff des Gerätes teleskopartig ineinander verschiebbar ein Innenrohr 27 und mindestens ein dieses umgreifendes Teleskoprohr 28 aufweist. Damit läßt sich das Universalgerät durch teleskopartiges Einschieben der einzelnen, den Griff bildenden Rohre auf geringe Abmessungen reduzieren, welche die Unterbringung erleichtern und gleichzeitig das Auskultieren vereinfachen.

Damit läßt sich ein Stethoskop so ausbilden, daß es bei geringen äußeren Abmessungen gleichzeitig als Reflexhammer verwendbar ist, eine zur Überprüfung von Reflexen dienende Nadel enthalten kann und mit ihm eine elektrische Lampe mit zugehöriger Batterie zur Verfügung stehen kann. Besonders vorteilhafte Ausgestaltungen des erfindungsgemäßen Steheskops werden in den abhängigen Ausprüchen angegeben.

Es hat sich einerseits als vorteilhaft erwiesen, den Griff fest mit dem Rumpf zu verbinden; andererseits werden sowohl der Transport als auch das Auskultieren vereinfacht, wenn der Griff durch einen Verschluß lösbar mit dem Rumpf verbunden ist, wobei ein einfacher, steckbarer Griff durch einen lösbaren Verschluß haltbar ist, der eine Rastverbindung aufweist oder als geringfügig konische Steckverbindung ausgeführt ist. Der Verschluß kann auch als Gewinde ausgeführt sein, das konisch angelegt oder als Steckgewinde ausgebildet sein kann. Hierbei ist der Griff mit dem Rumpf nur dann zu verbinden, wenn der Rumpf als Hammerkopf benötigt wird; bei der Verwendung als Stethoskop hingegen kann der Griff vom Rumpf entfernt und abgenommen oder über den Schlauch nach hinten geschoben werden. Eine einfache Anpassung an unterschiedliche Gegebenheiten läßt sich erreichen, wenn ein Bereich des Griffes als jeweils leicht zu verformender sogenannter Schwanenhals ausgebildet ist.

Die Einsatzmöglichkeiten des Stethoskopes lassen sich erweitern wenn zwei unterschiedliche Abhördosen bzw. -ausnehmungen vorgesehen sind; hierbei können zwar beide Ausnehmungen von Membranen überfangen sein, es hat sich jedoch bewährt, die Ausnehmung geringeren Durchmessers ohne Membran auszuführen und gegebenenfalls mit einem thermisch isolierenden Schutzring aus Kunststoff zu umgeben, während die Ausnehmung größeren Durchmessers durch eine Membran abgeschlossen ist oder eine mit einer Membran abgeschlossene Dose aufweist.

Ein sauberes Auskultieren unter Abschirmung vom Fremdgeräuschen läßt sich durchführen, wenn die am Grunde der Ausnehmungen vorgesehenen Bohrungen mittels eines Schalteinsatzes alternierend mit einer zu den Abhörschläuchen führenden Bohrung verbindbar sind, wobei zweckmäßig der Schalteinsatz in den alternativen Stellungen einrastet und seine Stellung durch leicht optisch erfaßbare Merkmale oder zusätzliche Konstruktionselemente anzeigt. Bewährt hat es sich, diesen Schalteinsatz auch gleichzeitig zur Aufnahme der Lichtquelle auszubilden und gegebenenfalls auch in einer weiteren, definierten Stellung einen Schalter zu betätigen.

Im einzelnen sind die Merkmale der Erfindung anhand der Beschreibung von Ausführungsbeispielen in Verbindung mit diese darstellenden Zeichnungen erläutert. Es zeigen hierbei:

Figur 1  perspektivisch den Rumpf eines Stethoskopes mit Schlauch und abgenommenem Griff, bei dem der Rumpf hammerkopfartig ausgebildet ist und in einem spiegelnden Reflektor eine Glühlampe aufweist,

Figur 2  das Gabelstück und den Hörteil des Stethoskops nach Fig. 1,

Figur 3  eine perspektivische Ansicht der Membranseite des Rumpfes des Stethoskops nach Fig. 1 mit eingeschraubtem Griff,

Figur 4  die Seitenansicht eines erfindungsgemäßen Stethoskops mit teleskopartig ausziehbarem Griff mit abgebrochen dargestellten Schläuchen,

Figur 5  die Ansicht des Stethoskops nach Fig. 4,

Figur 6  in größerem Maßstabe einen Längsschnitt durch den Rumpf des Stethoskops nach Fig. 4 mit abgebrochen dargestelltem Griffrohr,

Figur 7  einen Querschnitt durch den Rumpf des Stethoskops nach Fig. 4 ohne dessen Anbauteil,

Figuren 8  und 9 in den Rumpf des Stethoskops nach Fig. 4 bis 7 einsetzbare Hammereinsätze, und

Figur 10  eine Reflexnadel des Stethoskops nach Fig. 5.

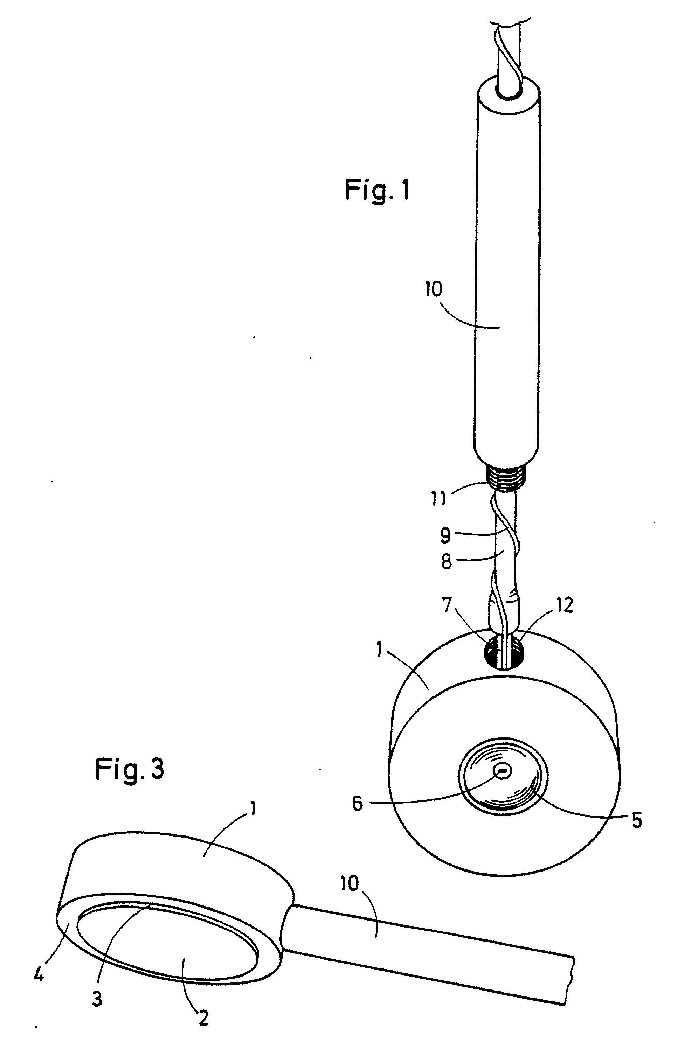

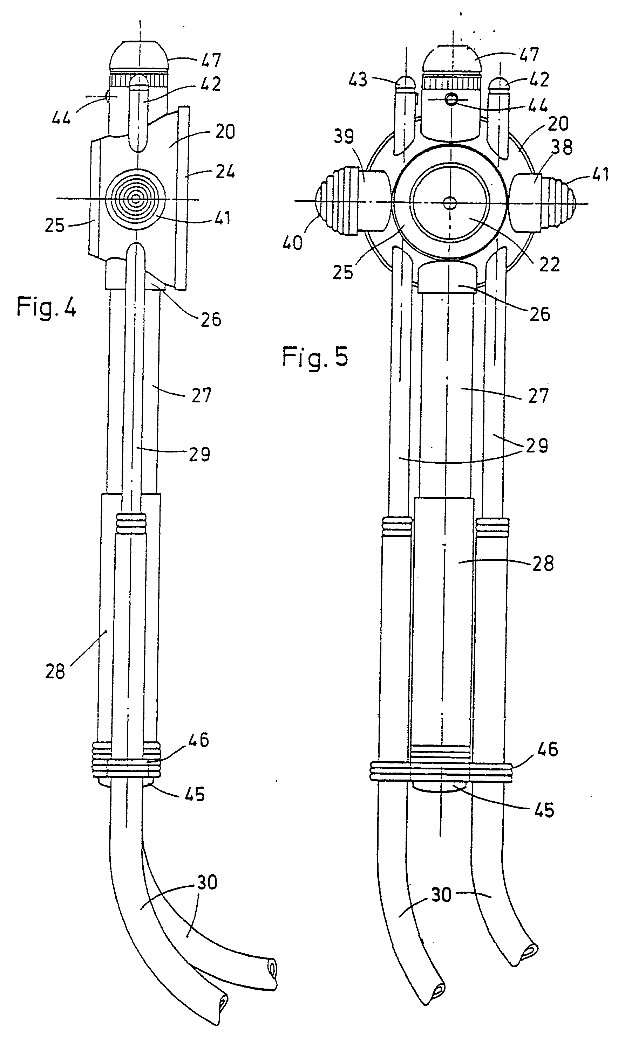

In den Fig. 1 und 3 ist ein Rumpf eines Stethoskops gezeigt, in dessen Ausnehmung 3 eine durch eine Membran abgeschlossene Dose eingesetzt ist, die von einer Umhüllung 4 umschlossen sind. Diese Umhüllung 4 besteht aus elastischem Material, bspw. Gummi oder Kunststoff. Sie kann, wie dargestellt, den Rumpf als Formstück vollständig umschließen oder aber als Gummiring mit bspw. kreisförmigem Querschnitt durch eine den Rumpf umschließende Nut gehalten werden.

In der oberen, der Membran 2 abgewandten Deckfläche weist der Rumpf 1 nach Fig. 1 eine Vertiefung 5 auf, in der eine Glühlampe 6, bspw. eine kleine Halogen-Glühlampe, eingelassen ist. Die Seitenwände der Vertiefung können hierbei paraboloidartig geformt und verspiegelt sein, so daß das Licht der Glühlampe 5 auch gerichtet wird, wenn keine Linsenlampe Verwendung findet.

Die durch die Membran 2 abgeschlossene Dose weist einen Anschlußstutzen 7 auf, auf den ein Schlauch 8 aufgesteckt ist. Um diesen Schlauch ist zur Stromversorgung der Lampe 6 ein zweiadriges Spiralkabel 9 gewickelt, das im Bereiche des Anschlußstutzens 7 in den Rumpf 1 ausgeführt ist. Über dem Schlauch 8 ist ein bspw. aus Kunststoff bestehender rohrförmiger Griff 10 geführt, der an seinem dem Rumpf 1 zugewandten Ende ein Gewinde 11 aufweist, das in ein entsprechendes Gegengewinde 12 des Rumpfes 1 schraubbar ist. Dadurch läßt sich der Griff 10 entsprechend der Darstellung der Fig. 3 mit dem Rumpf 1 verbinden, so daß dieser in Verbindung mit seiner Umhüllung 4 einen am Ende des Griffes 10 vorgesehenen Hammerkopf bildet. Der Griff 10 weist zweckmäßig in seinem Inneren in den Figuren nicht dargestellte Spreizfedern auf, die gegen den Schlauch 8 vorgespannt sind, so daß der Griff sich durch Reibung in der in Fig. 1 dargestellten Position zu halten vermag und der Rumpf 1 frei auf beliebige Körperpartien eines Patienten auflegbar ist, ohne daß das Griffrohr zu stören vermag.

Andererseits kann der Griff 10 aber auch mit dem Rumpf 1 festverbunden sein, und es ist auch möglich, den die Verbindung bewirkenden Verschluß nicht durch ein Schraubgewinde oder ein Renkgewinde bzw. einen Bajonettverschluß zu bilden, sondern der Griff kann sich auch konisch verjüngen und in eine entsprechende konische Aufnahme drehend einschieben. Auch eine zylindrische, mit einer Rastvorrichtung versehene oder mit hohem Reibwert fassende Aufnahme sind anwendbar. An die Stelle eines als Griff vorgesehenen Sternrohres kann auch ein Griff nach Art eines Schwanenhalses treten, der sich in gewünschte Formen biegen läßt.

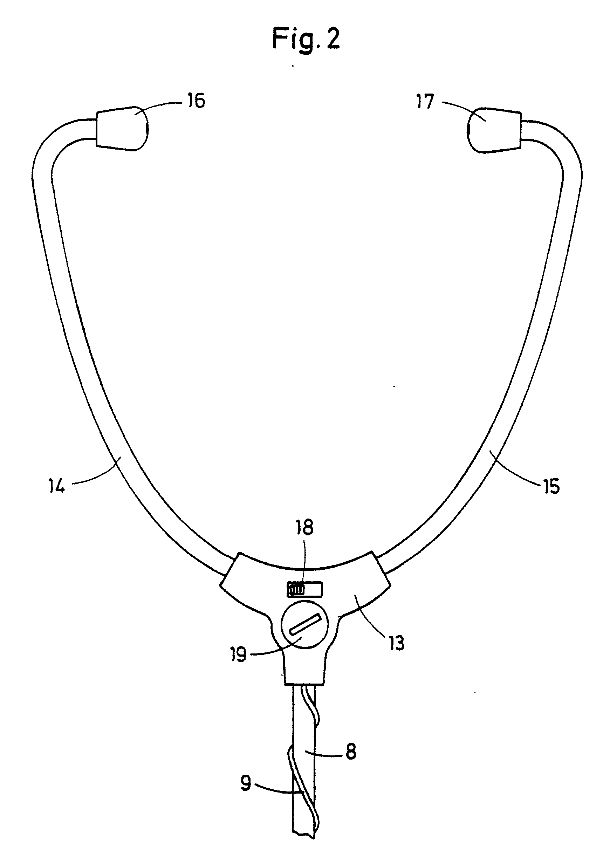

In der Fig. 2 ist ein an den Schlauch 8 der Fig. 1 angeschlossenes Gabelstück 13 dargestellt, durch welches der durch den Schlauch 8 zugeführte Schall auf zwei weitere Schläuche 14 und 15 verteilt wird, an deren freien Enden Ohroliven 16 und 17 zum Einführen der Schläuche in die Ohröffnungen des Diagnostizierenden vorgesehen sind. Das Gabelstück 13 ist weiterhin mit einem Schalter 18 und einem ein Batteriefach abschließenden Schraubverschluß 19 versehen. Das Batteriefach vermag eine Knopfbatterie zur Speisung der Glühlampe 6 der Fig. 1 aufzunehmen. Durch Betätigung des Schalters 18 wird ein Stromkreis von der Batterie über das Spiralkabel 9 und die Lampe 6 geschlossen, so daß die Glühlampe aufleuchtet. Der Schalter und/oder das Batteriefach lassen sich auch im Griff 10 oder aber im Rumpf 1 unterbringen.

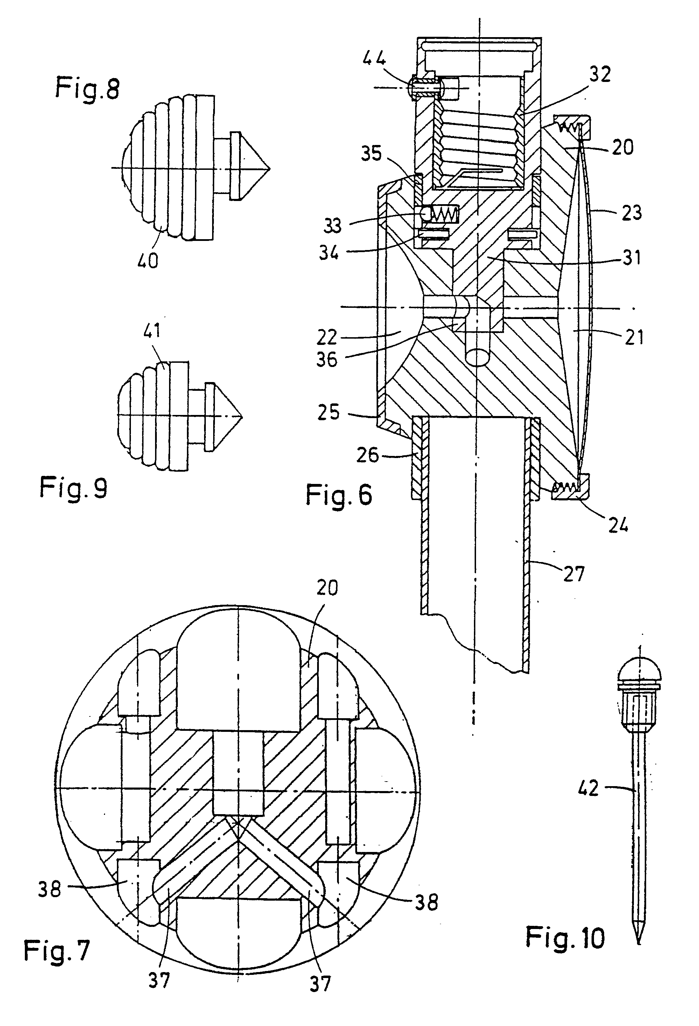

Ein Ausführungsbeispiel des erfindungsgemäßen Stethoskops ist in den Figuren 4 bis 10 dargestellt. Der Rumpf 20 dieses Stethoskops ist, wie insbesondere Fig. 6 zeigt, auf der Front- und Rückseite mit Ausnehmungen 21 und 22 unterschiedlichen Durchmessers ausgestattet. Die Ausnehmung 21 ist durch eine mittels eines Befestigungsringes 24 gehaltene Membran 23 überfangen, während bei der Ausnehmung 22 geringeren Durchmessers nur deren Randbereich durch einen aus Kunststoff bestehenden Schutzring 25 abgedeckt ist, um thermische Auswirkungen auf einen mit ihm in Berührung gebrachten Hautbereich zu begrenzen. In eine weitere Ausnehmung des Rumpfes 20 ist ein Befestigungsstutzen 26 eingesetzt, der ein Innenrohr 27 hält und gleichzeitig einen Anschlag für ein dieses gemäß Fig. 5 umschließendes Teleskoprohr 28 darstellt.

Die Abführung des aufgenommenen Schalls erfolgt über in den Rumpf 20 eingesetzte, dem Innenrohr 27 und dem Teleskoprohr 28 parallele Anschlußrohre 29, deren freie Enden von Schläuchen 30 übergriffen sind. Die Verbindung zu den am Grunde der Ausnehmungen 21 und 22 vorgesehenen Bohrungen erfolgt selektiv mittels eines in einer Ausnehmung des Rumpfes 20 drehbaren Schalteinsatzes 31, der gleichzeitig auch gemäß Fig. 6 eine Schraubfassung 32 für eine Glühlampe aufweist und gemäß Fig. 4 und 5 oberhalb der aufzunehmenden Glühlampe durch einen transparenten Aufsatz 27 abgeschlossen wird, dessen Stirnfläche zweckmäßig linsenartig ausgeführt ist. Drehbar gehalten wird der Schalteinsatz 31 durch einen in eine Nut desselben eingelegten Sicherungsring, der von den Stirnflächen eines in die Ausnehmung des Rumpfes 20 eingepreßten Raststutzens 35 übergriffen wird, der gleichzeitig seitliche Schlitze zum Einrasten einer federbelasteten Rastkugel 33 aufweist. Der Schaltansatz 36 des Schalteinsatzes 31 weist eine radiale, sich etwa bis zur Mitte erstreckende Bohrung auf, die in eine axiale Bohrung mündet. Damit läßt sich durch Drehen des Schalteinsatzes 31 um 180° alternierend die Schallöffnung der Ausnehmung 21 bzw. der Ausnehmung 22 mit den folgenden, in Fig. 7 dargestellten Bohrungen verbinden. Diese Bohrungen 37 sind von der dem Schaltansatz 36 zugewandten Stirnfläche der Ausnehmung des Rumpfes 20 bis zu achsparallelen, die Anschlußrohre 29 aufnehmenden Ausnehmungen 38 geführt, und die Anschlußrohre weisen im Mündungsbereich der Bohrungen 37 ihrerseits Eintrittsöffnungen auf.

Weitere, seitlich vorgesehene Ausnehmungen der Fig. 7 nehmen rohrförmige Sockel 39 auf, in die gemäß Fig. 5 in Figuren 8 und 9 gesondert dargestellte Hammeraufsätze 40 und 41 so eingedrückt sind, daß ihre hinterschnittenen Fußzapfen sich festsetzen.

Entsprechend den Fig. 4 und 5 sind weitere Ausnehmungen der Fig. 7 mit rohrartigen Ansätzen bestückt, deren einer eine in Fig. 10 dargestellte Reflexnadel aufnimmt, während die andere mit einer seitlich auskragenden Schaltfeder bestückt ist.

Wird der Schalteinsatz 31 so gedreht, daß der die aus Kunststoff bestehende Schraubfassung 32 haltende Niet 44 dieser Feder des Schalters 43 der Fig. 5 gegenübersteht, so wird der Niet 44 unter Spannung gesetzt und gibt diese durch die mit seiner Rückseite verbundene Feder auf den Sockel der von der Schraubfassung 32 aufzunehmenden Glühlampe weiter, deren zentraler Anschlußpunkt auf der mit Masse verbindenden, die Schraubfassung 32 unterfangenden Feder aufliegt, so daß eine eingebrachte Glühlampe aufleuchtet.

Eine kleine Knopfzelle könnte in einem im Rumpf 20 vorgesehenen Batteriefach untergebracht werden. Eine höhere Anzahl von Betriebsstunden und damit ein verläßlicheres Arbeiten in Verbindung mit der Vermeidung von Spezialbatterien läßt sich erzielen, wenn das Innenrohr 27 als Batterieaufnahme ausgebildet wird. Nach eingeschobenem Teleskoprohr 28 und Abnehmen des Verschlußstopfes 45 wird dann das Batteriefach erreichbar, und die Batterie läßt sich nach Entnehmen eines das Innenrohr 27 nach unten abschließenden Deckels, der als Schraubverschluß ausgebildet sein kann, austauschen.

Die Schläuche 30 sind zur Entlastung ihrer Anschlußstelle und zur Vermeidung scharfer Knicke durch einen brillenartig ausgebildeten Schlauchhalter 46 geführt, der fest auf dem unteren Ende des Teleskoprohres 28 angeordnet ist. Damit läßt sich durch einfaches Abziehen, gegebenenfalls in Verbindung mit Drehen, dem Gerät die Reflexnadel 42 entnehmen. Dem Untersuchenden stehen zwei unterschiedlich geformte Hammeraufsätze 40 und 41 zur Verfügung, die er auch bei Benutzung als Stethoskop, zweckmäßig nach Aufschieben des Teleskoprohres 28, die Wahl zwischen zwei unterschiedlichen Schallaufnahmeöffnungen hat, wobei Fremdgeräusche der jeweils nicht benutzten Ausnehmung durch Betätigen des Schalteinsatzes 31 ausgeschaltet werden, der gleichzeitig auch das Einschalten einer Glühlampe bewirkt, die über einen frontseitig linsenartig gestalteten Aufsatz 47 scharf gebündeltes Licht abzugeben vermag, nach Abnehmen des Aufsatzes stärker divergierendes Licht, und gegebenenfalls unter Benutzung einer Streuscheibe diffuses Licht vermittelt.

In allen diesen Fällen benötigt der Arzt für seine Visiten oder Krankenbesuchte nunmehr nur ein einziges Gerät, das gleichzeitig zum Auskultieren, zum Ausleuchten und zum Feststellen von Reflexen dienen kann. Er kann daher stets sicher sein, die jeweils wichtigsten Geräte für allgemeine Untersuchungen mitzuführen und gegen Verlust, Verlegen, Verlieren oder dergleichen derselben gesichert zu sein.

**Patentansprüche**

**1.** Stethoskop mit einem Rumpf (20) mit mindestens einer trichterförmigen Ausnehmung (21, 22) um Erfassen des Schalles und mit zwei mit dem Grunde der Ausnehmung in Verbindung stehenden, mit Ohroliven versehenen Schläuchen (30), bei dem der Rumpf durch eine elastische Umhüllung (4) oder durch mindestens einen elastischen Hammeraufsatz (40, 41) zu einem Hammerkopf ausgebildet ist, der mit einem rohrförmigen Griff verbunden oder verbindbar ist,
**dadurch gekennzeichnet,**
daß der Griff teleskopartig ineinander verschiebbar ein Innenrohr (27) und mindestens ein dieses umgreifendes Teleskoprohr (28) aufweist.

**2.** Stethoskop nach Anspruch 1,
**dadurch gekennzeichnet,**
daß einander gegenüberliegende Stirnflächen des Rumpfes (20) jeweils mit einer den Schall aufnehmenden Ausnehmung (21, 22) unterschiedlicher Durchmesser versehen sind, deren Grund jeweils bis zu einer Ausnehmung des Rumpfes geführt ist, in der drehbar ein Schalteinsatz (31) vorgesehen ist, der eine radiale, in eine axiale Ausgangsbohrung einmündende Eingangsbohrung aufweist, und daß die Ausgangsbohrung des Schalteinsatzes (31) in Verbindung mit den Schläuchen (30) steht, und daß die Eingangsbohrung durch Drehen des Schalteinsatzes (31) jeweils alternierend unter eine der Ausnehmungen (21, 22) bringbar ist.

**3.** Stethoskop nach Anspruch 2,
**dadurch gekennzeichnet,**
daß der Schalteinsatz (31) eine Fassung (32) einer Glühlampe aufweist und ihm ein dieser vorgeordneter elektrischer Schalter zugeordnet ist, daß das Innenrohr (27) des Griffes als Batteriefach ausgebildet ist, und daß dem Schalteinsatz (31) eine die eingestellte seiner Betriebslagen sichernde Rastvorrichtung (Rastkugel (33)) zugeordnet ist.

**Claims**

**1.** A stethoscope having a trunk (20) with at least one funnel-shaped recess (21, 22) for detecting sound and with two tubes (30) which are connected to the base of the recess and are provided with ear tips, in which the trunk is supplemented by an elastic sheathing (4) or by at least one elastic hammer attachment (40, 41) to form a hammer head, which is connected or can be connected with a tubular grip,
characterised in that the grip comprises an inner tube (27) and at least one telescopic tube (28) surrounding it, which are telescopically displaceable inside one another.

**2.** A stethoscope according to Claim 1,
characterised in that front surfaces of the trunk (20) opposite one another are respectively provided with a recess (21, 22) having a variable diameter and receiving the sound, the base of which is respectively guided to a recess in the trunk, in which a switch bushing (31), which comprises a radial inlet bore opening into an axial outlet bore, is rotatably provided,
and in that the outlet bore of the switch bushing (31) is connected with the tubes (30),
and in that the inlet bore can be alternately moved under one of the recesses (21, 22) by rotating the switch bushing (31).

**3.** A stethoscope according to Claim 2,
characterised in that the switch bushing (31) comprises a socket (32) for an electric bulb and an electric switch connected at the inlet side is associated thereto,
in that the inner tube (27) of the grip is constructed as a battery housing,
and in that a stop device (stop ball 33) ensuring its respective operating position is associated with the switch bushing (31).

**Revendications**

**1.** Stéthoscope comprenant un corps (20) comportant au moins un évidement (21,22) en forme d'entonnoir pour la réception du son et deux tuyaux souples (30) reliés au fond de l'évidement et munis d'olives auriculaires, dans lequel le corps est constitué par une enveloppe élastique (4) ou par au moins une garniture de marteau élastique (40,41) de manière à réaliser une tête de marteau qui est reliée ou peut être reliée à un manche de forme tubulaire, caractérisé en ce que le manche comporte, montés de manière à pouvoir se déplacer l'un dans l'autre, un tube intérieur (27) et au moins un tube télescopique (28) qui l'entoure.

**2.** Stéthoscope selon la revendication 1, caractérisé en ce que les surface opposées du corps (20) comportent chacune, pour la réception du son, un évidement (21,22) de diamètre différent dont le fond rejoint un évidement du corps dans lequel est montée, de manière à pouvoir tourner, une pièce de commutation (31) qui comporte un alésage d'entrée radial débouchant dans un alésage de sortie axial, en ce que l'alésage de sortie de la pièce de commutation (31) est relié aux tuyaux souples (30) et en ce que la rotation de la pièce de commutation (31) permet de placer alternativement l'alésage d'entrée au-dessous de l'un des évidements (21,22).

3. Stéthoscope selon la revendication 2, caractérisé en ce que la piéce de commutation (31) comporte une douille (32) d'une lampe à incandescence et est associée à un commutateur électrique situé en amont, en ce que le tube intérieur (27) du manche constitue l'enveloppe d'une pile électrique et qu'à la pièce de commutation (31) est associé un dispositif d'arrêt (bille d'arrêt (33)) qui la bloque dans l'une de ses positions de fonctionnement.

Fig. 1

10

11

9

8

7    12

1

6    5

Fig. 3

1

10

4

3    2

# Fig. 2

Fig. 4

Fig. 5

Fig.8

Fig.9

Fig. 6

Fig.7

Fig.10